(19) Europäisches Patentamt
European Patent Office
Office européen des brevets

(11) Numéro de publication : **0 040 591**
**B1**

(12) **FASCICULE DE BREVET EUROPÉEN**

(45) Date de publication du fascicule du brevet : **22.06.83**

(51) Int. Cl.³ : **C 07 D213/66**, A 61 K 31/44

(21) Numéro de dépôt : **81870023.9**

(22) Date de dépôt : **15.05.81**

(54) **Dérivés de pyridoxine, leur procédé de préparation ainsi que leur utilisation en thérapeutique.**

(30) Priorité : **19.05.80 GB 8016516**

(43) Date de publication de la demande :
**25.11.81 Bulletin 81/47**

(45) Mention de la délivrance du brevet :
**22.06.83 Bulletin 83/25**

(84) Etats contractants désignés :
**AT BE CH DE FR IT LI LU NL SE**

(56) Documents cités :
**EP A 0 006 614**
**FR A 2 218 101**
**FR A 2 383 930**
**US A 4 065 461**

(73) Titulaire : **S.A. LABAZ - SANOFI N.V.**
**Avenue De Béjar, 1**
**B-1120 Bruxelles (BE)**

(72) Inventeur : **Descamps, Marcel**
**Rue du Bois du Bosquet, 30**
**B-1331 Rosieres (BE)**
Inventeur : **Urbain, Marcel**
**Decede (BE)**

(74) Mandataire : **Cauchie, Daniel et al**
**c/o S.A. LABAZ-SANOFI N.V. Avenue De Béjar, 1**
**B-1120 Bruxelles (BE)**

**EP 0 040 591 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

Dérivés de pyridoxine, leur procédé de préparation ainsi que leur utilisation en thérapeutique

La présente invention se rapporte à de nouveaux dérivés de pyridine en particulier à de nouveaux dérivés de pyridoxine ainsi qu'à leur procédé de préparation.

Les dérivés de pyridoxine de l'invention peuvent être représentés par la formule générale :

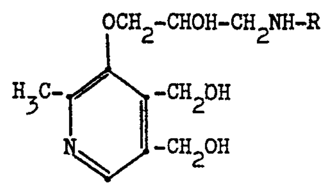

dans laquelle R représente un radical

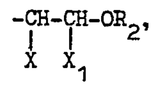 ou $-CH_2-(CH_2)_n-CH_2-OR_2$

dans lequel n représente 0 ou 1, X et $X_1$, qui sont différents, représentent hydrogène ou méthyle, et $R_2$ représente un groupement phényle non substitué ou substitué par un ou deux substituants sélectionnés du groupe formé par le fluor, le chlore et le brome et par les radicaux méthyle, éthyle, n-propyle, isopropyle et méthoxy.

L'invention se rapporte également aux sels d'addition non toxiques des composés de formule I, par exemple les sels obtenus au départ de l'acide chlorhydrique, l'acide oxalique, l'acide fumarique, l'acide maléique et l'acide pamoïque.

L'invention se rapporte notamment aux dérivés de pyridoxine de formule générale I dans laquelle X, $X_1$ et n ont la signification y citée et $R_2$ représente un groupement phényle non substitué ou substitué par un ou deux radicaux méthyle ou encore par un radical éthyl-2, n-propyl-2, isopropyl-2 ou méthoxy-2 phényle, diméthoxy-3,4 phényle, isopropyl-2 méthyl-5 phényle ou chloro-4 phényle, ainsi que les sels d'addition non toxiques de ces composés.

Comme exemples de tels dérivés de pyridoxine, on peut citer les composés suivants :

Dihydroxyméthyl-4,5[hydroxy-2(méthyl-2 phénoxyéthylamino)-3 propoxy]-3 méthyl-2 pyridine
Dihydroxyméthyl-4,5(hydroxy-2 phénoxyéthylamino-3 propoxy)-3 méthyl-2 pyridine
Dihydroxyméthyl-4,5[hydroxy-2(méthyl-3 phénoxyéthylamino-3 propoxy]-3 méthyl-2 pyridine
Dihydroxyméthyl-4,5[(éthyl-2 phénoxyéthylamino)-3 hydroxy-2 propoxy]-3 méthyl-2 pyridine
Dihydroxyméthyl-4,5[(diméthyl-2,6 phénoxyéthylamino)-3 hydroxy-2 propoxy]-3 méthyl-2 pyridine
Dihydroxyméthyl-4,5[hydroxy-2(diméthyl-3,5 phénoxyéthylamino)-3 propoxy]-3 méthyl-2 pyridine
Dihydroxyméthyl-4,5[hydroxy-2(méthoxy-2 phénoxyéthylamino)-3 propoxy]-3 méthyl-2 pyridine
Dihydroxyméthyl-4,5[hydroxy-2(phénoxy-2 méthyl-2 éthylamino)-3 propoxy]-3 méthyl-2 pyridine
Dihydroxyméthyl-4,5[hydroxy-2(méthyl-1 phénoxy-2 éthylamino)-3 propoxy]-3 méthyl-2 pyridine
Dihydroxyméthyl-4,5[hydroxy-2(isopropyl-2 méthyl-5 phénoxyéthylamino)-3 propoxy]-3 méthyl-2 pyridine
Dihydroxyméthyl-4,5[hydroxy-2(méthyl-4 phénoxyéthylamino)-3 propoxy]-3 méthyl-2 pyridine
Dihydroxyméthyl-4,5[hydroxy-2(n-propyl-2 phénoxyéthylamino)-3 propoxy]-3 méthyl-2 pyridine
Dihydroxyméthyl-4,5[hydroxy-2(isopropyl-2 phénoxyéthylamino)-3 propoxy]-3 méthyl-2 pyridine
Dihydroxyméthyl-4,5[hydroxy-2 (diméthyl-3,4 phénoxypropylamino)-3 propoxy]-3 méthyl-2 pyridine
Dihydroxyméthyl-4,5[hydroxy-2(chloro-4 phénoxyéthylamino)-3 propoxy]-3 méthyl-2 pyridine

ainsi que leurs sels d'addition non toxiques par exemple ceux obtenus à partir de l'acide chlorhydrique, l'acide oxalique, l'acide fumarique, l'acide maléique et l'acide pamoïque.

Les composés de formule I ci-dessus présentent dans la chaîne située en position 3 un ou plusieurs carbones asymétriques et peuvent donc se trouver sous la forme d'isomères optiques, d'isomères de position ou de mélanges de ces isomères. Si on le désire, on peut séparer ces isomères de leur mélange aux étapes appropriées de la préparation de ces mélanges par des méthodes connues en soi pour obtenir les isomères individuels désirés. L'invention se rapporte à la fois aux isomères individuels en question et aux mélanges racémiques de ces isomères.

On a découvert que les dérivés de pyridoxine de l'invention possèdent de remarquables propriétés pharmacologiques susceptibles de les rendre utiles dans le traitement de l'hypertension artérielle, de l'angine de poitrine ou dans le traitement des déficits circulatoires au niveau du cerveau ou du myocarde.

Un autre objet de l'invention concerne des compositions pharmaceutiques ou vétérinaires comprenant, comme principe actif, au moins un dérivé de pyridoxine de formule I ou un sel d'addition non toxique de ce dérivé en association avec un véhicule pharmaceutique ou un excipient approprié.

De même, l'invention se rapporte à un procédé de préparation de compositions pharmaceutiques ou vétérinaires, procédé suivant lequel on associe au moins un dérivé de pyridoxine de formule I ou un sel d'addition non toxique de ce dérivé avec un véhicule pharmaceutique ou un excipient approprié.

Les composés de formule I peuvent être préparés au départ d'α,α-isopropylidènepyridoxol de formule :

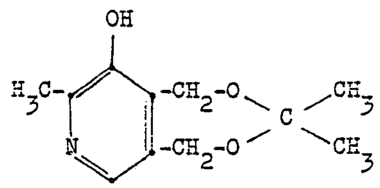

II

ou d'un sel de ce composé par exemple le chlorhydrate.

Le composé de formule II en question est un produit connu lequel a été décrit par W. Korytnyk dans J. Org. Chem. 1962, *27*, 3724.

On condense tout d'abord le dérivé diméthylcétal de formule II avec le chlorure d'allyle et ce, au reflux, dans un solvant approprié tel qu'un alcool inférieur de 1 à 3 atomes de carbone, par exemple le méthanol ou l'éthanol et en présence de sodium pour donner le O-allyl-3 α,α-isopropylidènepyridoxol de formule :

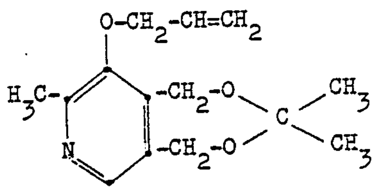

III

que l'on fait réagir, par la suite, à la température ambiante avec la N-bromosuccinimide en présence d'eau et dans un solvant approprié par exemple le benzène, l'éther diéthylique ou l'éther diisopropylique, pour obtenir un mélange de deux bromhydrines lequel après traitement au moyen d'hydroxyde de sodium fournit le O-(époxy-2,3 propyl)-3 α,α-isopropylidènepyridoxol de formule :

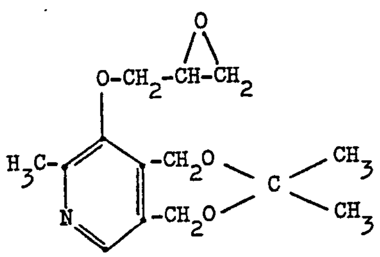

IV

On condense alors l'époxyde de formule IV au reflux et dans un solvant approprié tel qu'un alcool inférieur de 1 à 3 atomes de carbone par exemple le méthanol avec une amine primaire de formule générale :

$$H_2N—R \qquad V$$

dans laquelle R a la même signification que précédemment.

On procède ensuite à l'hydrolyse du cétal intermédiaire ainsi obtenu et ce, en milieu acide fort par exemple acide chlorhydrique et à une température comprise entre 25 et 80 °C, pour obtenir les composés désirés sous forme de base libre.

Les sels d'addition non toxiques de l'invention pourront être obtenus, de manière classique, en faisant réagir le dérivé de pyridoxine correspondant de formule I avec un acide inorganique approprié tel que par exemple l'acide chlorhydrique ou un acide organique approprié tel que par exemple l'acide oxalique, l'acide fumarique, l'acide maléique ou l'acide pamoïque. On a décrit dans la demande de brevet français n° 2 218 101, des dérivés de pyridine lesquels sont présentés comme possédant une activité bloquante vis-à-vis des récepteurs β-adrénergiques. De même, on a décrit dans le brevet belge n° 864 917, des dérivés pyridiniques en tant qu'agents anti-arythmiques et/ou anesthésiques locaux.

Ces brevets couvrent un nombre extrêmement élevé de produits finaux et incluent certains dérivés de

pyridoxine de la présente invention uniquement par la combinaison des divers substituants envisagés sur le radical pyridine. Toutefois, aucun dérivé comportant la structure caractéristique de la pyridoxine, et à fortiori aucun dérivé de la présente invention, n'y a été spécifiquement cité ou décrit.

Parmi les dérivés pyridiniques de l'art antérieur précité sont mentionnés des dérivés comportant un radical hydroxy-2 amino-3 propoxy N-substitué. Il est remarquable de constater que parmi les nombreuses valeurs attribuées au substituant situé au niveau de l'atome d'azote de ce radical, seuls des radicaux alkyles ont été exemplifiés à savoir le radical t-butyle et, dans la grande majorité des cas, le radical isopropyle.

Au cours de l'élaboration de la présente invention, des essais ont été effectués au moyen de dérivés de pyridoxine comportant en position 3 une chaîne hydroxy-2 amino-3 propoxy substituée sur l'atome d'azote par un seul radical alkyle en l'occurrence un radical n-propyle, isopropyle, méthyl-1 propyle, isobutyle ou t-butyle ou encore par un radical phénoxyalkyle en l'occurrence (méthoxy-2 phénoxy)-éthyle ou (méthyl-2 phénoxy)-éthyle. De tels essais avaient pour but de vérifier les propriétés pharmacologiques attribuées aux composés non décrits dans ces susdits brevets. Cependant, les dérivés N-alkyles en question n'ont pas montré les propriétés β-bloquantes attendues ni même aucunes propriétés antiadrénergiques de type α ou β. Dans le cas des dérivés phénoxyéthyles aucunes propriétés antiarythmiques ou anesthésiques locales n'ont pu être mises en évidence de manière significative.

Quant à la demande de brevet européen n° 0 006 614, celle-ci se rapporte notamment à des dérivés de pyridine comportant un radical hydroxy-2 amino-3 propoxy, N-substitué par un radical phénylalkyle ou phénoxyalkyle, l'entité phényle étant substituée ou non. Ces composés dont le noyau pyridyle ne comporte aucun autre substituant sont présentés comme agents antiadrénergiques β, certains d'entre eux ayant également des propriétés antiadrénergiques α. Il a maintenant été trouvé que des dérivés de pyridine non décrits dans les susdits brevets et formés au départ de l'entité méthyl-2 dihydroxyméthyl-4,5 pyridinyl-3 non décrite spécifiquement dans chacun de ces brevets et comportant un radical hydroxy-2 amino-3 propoxy N-substitué par un radical phénoxyalkyle présentent des propriétés pharmacologiques totalement imprévisibles de celles de l'état de la technique.

Ainsi, on a trouvé que la combinaison de l'entité méthyl-2 dihydroxyméthyl-4,5 pyridinyl-3 avec un radical hydroxy-2 phénoxyalkylamino-3 propoxy donne naissance à des composés ayant en commun de puissantes propriétés antiadrénergiques de type α, la plupart d'entre eux présentant en outre d'intéressantes propriétés antiadrénergiques vis-à-vis des récepteurs β.

Il a été constaté, par ailleurs de manière surprenante, que ces dérivés hydroxy-2 phénoxyalkylamino-3 propoxy sont plus actifs comme agents antiadrénergiques α que les dérivés hydroxy-2 phénylalkylamino-3 propoxy correspondants. De plus, certains composés de l'invention présentent un effet hypotenseur net chez l'animal normotendu anesthésié qui se traduit par un effet antihypertenseur chez l'animal vigile hypertendu. Cet effet est probablement consécutif à l'activité antiadrénergique α qui permet une diminution des résistances périphériques et en partie, probablement aussi, à une activité de type réserpinique. Comme la stimulation des récepteurs adrénergiques par les catécholamines endogènes aboutit à une augmentation du travail cardiaque et à une augmentation encore plus importante de la consommation énergétique cardiaque, il est reconnu qu'une médication ayant des propriétés antiadrénergiques peut se révéler antiangineuse en protégeant le cœur contre l'activité hypermétabolisante des catécholamines. Les composés de l'invention doués d'une activité antiadrénergique α et β seront donc aussi indiqués dans le traitement de l'angor. Leur intérêt est encore plus marqué dans cette indication car ils diminuent le travail du myocarde puisqu'ils provoquent une baisse de la pression artérielle et donc une diminution de la résistance à l'éjection cardiaque.

De plus, on connaît l'intérêt des composés ayant une activité antiadrénergique α, comme les dérivés hémisynthétiques de l'ergot de seigle, notamment la nicergoline, pour traiter un certain nombre de troubles cérébraux liés à un désordre d'origine vasculaire, en particulier l'insuffisance circulatoire cérébrale et la migraine.

Cet effet est d'autant plus intéressant chez les composés de l'invention qu'il est lié à un léger effet dépresseur central qui devrait être accompagné d'une diminution du métabolisme cérébral.

Les composés de l'invention les plus intéressants dans le traitement de l'insuffisance cérébrale seront ceux qui présentent les effets antiadrénergiques les plus puissants avec les effets hypotenseurs les moins importants.

Enfin, on a trouvé de manière surprenante que les composés de la présente invention présentent un très faible degré de toxicité, que ce soit par voie intraveineuse ou orale, ce qui leur confère un index toxico-pharmacologique très favorable et en particulier bien supérieur à celui des composés actuellement utilisés en thérapeutique pour les mêmes indications. Parmi les composés de l'invention ayant montré le spectre d'activité pharmacologique le plus intéressant, on peut citer :

— la dihydroxyméthyl-4,5[hydroxy-2(méthyl-2 phénoxyéthylamino)-3 propoxy]-3 méthyl-2 pyridine et

— la dihydroxyméthyl-4,5[hydroxy-2(méthoxy-2 phénoxyéthylamino)-3 propoxy]-3 méthyl-2 pyridine, ces produits étant sous forme de base libre ou de sels d'addition non toxiques tels que l'oxalate, le fumarate ou le dichlorhydrate.

Des tests pharmacologiques effectués avec la dihydroxyméthyl-4,5-[hydroxy-2(méthyl-2 phénoxyéthylamino)-3 propoxy]-3 méthyl-2 pyridine sous forme de son sesquioxalate (ci-après dénommé

Composé A) ont montré que ce composé présente, après administration par voie intraveineuse chez le chien, des propriétés α-antiadrénergiques dès la dose de 2 mg/kg et des propriétés antihypertensives dès la dose de 0,1 à 0,2 mg/kg.

De même, par voie intraveineuse chez le chien, la dihydroxyméthyl-4,5-[hydroxy-2(méthoxy-2 phénoxyéthylamino)-3 propoxy]-3 méthyl-2 pyridine sous forme de son dichlorhydrate (ci-après dénommé Composé φ) a montré une activité α-antiadrénergique prolongée dès la dose de 0,5 mg/kg. Les résultats de tests pharmacologiques entrepris avec les composés de l'invention en vue de mettre en évidence leurs effets cardiovasculaires et dépresseurs du système nerveux central sont cités ci-après.

A. Propriétés antiadrénergiques

Le but de ce test est de déterminer la capacité des composés à étudier à réduire l'augmentation de pression artérielle due à l'épinéphrine (effet anti-α) et l'accélération cardiaque due à l'épinéphrine (effet anti-β) chez le chien anesthésié au préalable avec du pentobarbital (30 mg/kg) et atropiné (1 mg/kg).

— Effet anti-α

Chez chaque chien, on détermine d'abord la dose d'épinéphrine qui provoque une augmentation reproductible d'environ 100 mmHg de la pression artérielle (entre 5 et 10 μg/kg).

Après quoi, on administre la dose d'épinéphrine ainsi déterminée suivie d'une dose, par voie intraveineuse, du composé à étudier. On enregistre alors le pourcentage de réduction de l'hypertension provoquée par le composé à étudier en comparaison avec l'hypertension obtenue précédemment (environ 100 mm Hg).

— Effet anti-β

Durant le même test que celui décrit ci-dessus, l'épinéphrine provoque une augmentation reproductible de la fréquence cardiaque d'environ 70 battements/min.

Le pourcentage de réduction de la fréquence cardiaque provoquée par le composé à étudier comparativement à la tachycardie mesurée précédemment (environ 70 battements) est alors enregistrée.

Dans les deux cas, les résultats ont été exprimés comme suit :

+ pour une réduction de l'augmentation de la pression ou de la fréquence cardiaque < 50 % ;

++ pour une réduction de l'augmentation de la pression ou de la fréquence cardiaque ⩾ 50 % ;

+++ pour une réduction subtotale de l'augmentation de la pression ou de la fréquence cardiaque.

Les résultats suivants ont été enregistrés avec des composés de formule I sous forme de sel avec l'acide oxalique.

| R | Dose (mg/kg) | Effet anti-α | Effet anti-β | Composé |
|---|---|---|---|---|
| 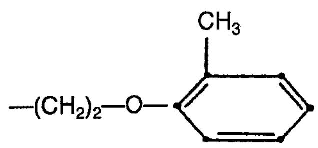 | 10 | +++ | + | A |
| 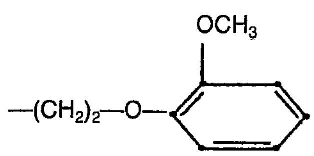 | 3,5 | +++ | +++ | B |
| 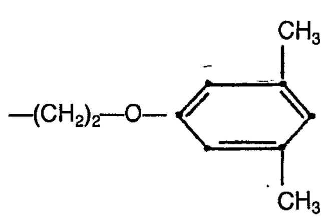 | 10 | +++ | + | C |
| 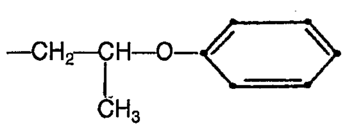 | 10 | +++ | ++ | D |

(Suite)

| R | Dose (mg/kg) | Effet anti-α | Effet anti-β | Composé |
|---|---|---|---|---|
| 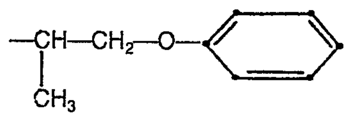 | 7,5 | +++ | + | E |
| 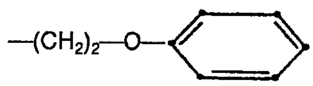 | 2 | +++ | ++ | F |
| 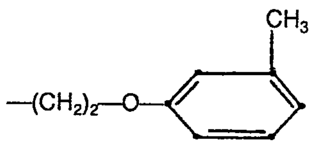 | 10 | ++ | + | G |
| 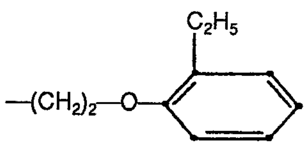 | 10 | +++ | + | H |
| 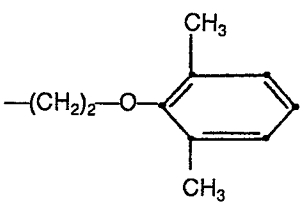 | 19 | ++ | ++ | I |
| 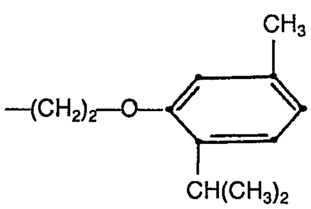 | 10 | +++ | ++ | J |
| 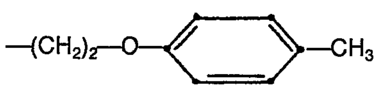 | 10 | ++ | 0 | K |
| 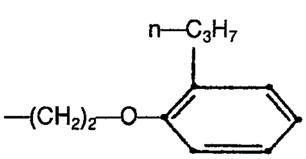 | 7 | +++ | ++ | L |
| 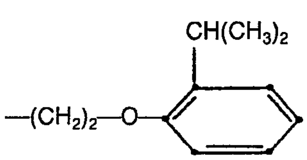 | 2 | +++ | ++ | M |
| 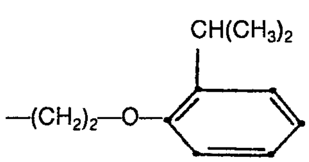 | 10 | +++ | ++ | N (*) |

(Suite)

| R | Dose (mg/kg) | Effet anti-α | Effet anti-β | Composé |
|---|---|---|---|---|
| 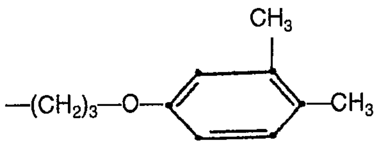 | 10 | ++ | ++ | P |
| 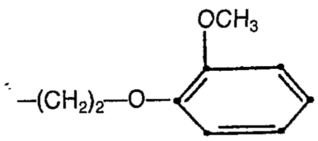 | 10 | +++ | ++ | φ (**) |
| 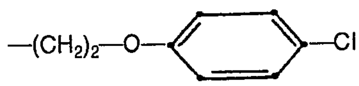 | 10 | ++ | + | R |

(*) utilisé sous forme de base
(**) utilisé sous forme de dichlorhydrate.

B. Propriétés antihypertensives

a) Hypotension chez le chien anesthésié
L'effet hypotenseur a été jugé pendant au moins un heure après l'injection intraveineuse d'une dose de composé à étudier et ce, chez le chien anesthésié au pentobarbital (30 mg/kg) et atropiné (1 mg/kg).
La pression artérielle a été captée au niveau d'une artère fémorale. Les résultats ont été exprimés comme suit :
++ pour une diminution durable d'environ 30 % de la pression artérielle ;
+ pour une diminution passagère d'environ 20 % de la pression artérielle. On a enregistré les résultats suivants avec les composés précités :

| Composé | Dose (mg/kg) | Diminution de la pression artérielle |
|---|---|---|
| A | 2 | ++ |
| B | 3,5 | + |
| E | 10 | + |
| G | 2 | ++ |

b) Hypotension chez le rat Okamoto
Dans cet essai, on a utilisé des rats de souche Okamoto dont la pression artérielle au début de l'essai se situe aux environs de 180 mm Hg et auxquels on administre une dose du composé à étudier.
La diminution de la pression artérielle a été enregistrée toutes les heures pendant 6 heures après l'administration du composé en question. Par voie orale, le produit de l'invention qui s'est révélé le plus actif en traitement aigu est le Composé A ci-dessus qui se révèle hypotenseur dès la dose de 25 mg/kg provoquant une diminution maximale de 21 mm Hg de la pression artérielle.
En traitement chronique, à raison d'une seule administration journalière le Composé A, s'est révélé actif dès la dose de 50 mg/kg.

C. Propriétés de type réserpinique

Celles-ci ont été jugées, après injections intraveineuses successives de 1, 2 et 4 mg/kg du composé à étudier chez le chien anesthésié, en déterminant les paramètres suivants :
— l'augmentation de la fréquence cardiaque,
— l'augmentation de dP/dt maximum, c'est-à-dire du maximum de la dérivée de la pression ventriculaire en fonction du temps. Cette dérivée représente donc un index de contractilité.
Les résultats montrent que les Composés A, C, E, H, J, K et M provoquent une augmentation de 10 à

30 % de la fréquence cardiaque et de la contractilité myocardique à la dose de 1 mg/kg.

Pour des doses supérieures, les effets indiqués ci-dessus ne sont pas plus importants et peuvent même être inversés.

Ces effets sont supprimés chez le chien préalablement réserpiné notamment lorsqu'on utilise le Composé A.

Par conséquent, ces effets sont dus à une libération des catécholamines hors des granules de stockage comme avec les réserpiniques conduisant ainsi à un effet antihypertenseur.

D. Propriétés dépressives centrales

Trente minutes après administration intragastrique d'une dose du composé à étudier, on place des groupes de souris dans une cage traversée de part en part par un rayon lumineux. Au moyen d'une cellule photoélectrique, on enregistre pendant 15 minutes, le nombre de passages des animaux au travers du rayon lumineux. Parallèlement, on effectue le même test sur des souris témoins.

On peut ainsi calculer la $DA_{50}$ c'est-à-dire la dose du composé à étudier qui provoque une action dépressive chez 50 % des animaux.

On a enregistré les résultats suivants :

| Composé | $DA_{50}$ en mg/kg |
|---|---|
| A | 50 |
| G | 33 |
| H | 47 |
| I | 44 |
| J | 40 |
| L | 42 |
| M | 42 |

E. Toxicité aiguë

Des tests de toxicité aiguë ont été pratiqués chez le rat et la souris avec des composés de l'invention.

On a enregistré les résultats suivants avec les composés ci-dessous :

I)

| R | $R_1$ |
|---|---|
| 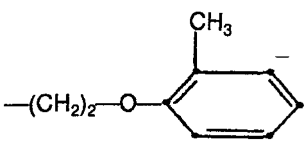 | H |

a) Sous forme de sesquioxalate

1) Chez la souris
- — Par voie intragastrique : $DL_{50}$ = 700 mg/kg
- — Par voie intrapéritonéale : $DL_{50}$ = 290 mg/kg
- — Par voie intraveineuse : $DL_{50}$ = 118 mg/kg

2) Chez le rat
- — Par voie intragastrique : $DL_{50}$ = 2 200 mg/kg
- — Par voie intrapéritonéale : $DL_{50}$ = 320 mg/kg
- — Par voie intraveineuse : $DL_{50}$ = 170 mg/kg

b) Sous forme de fumarate

1) Chez le rat
- — Par voie intragastrique : $DL_0$ > 2 500 mg/kg
- — Par voie intraveineuse : $DL_{50}$ = 200 mg/kg

(II)

| R | $R_1$ |
|---|---|
| 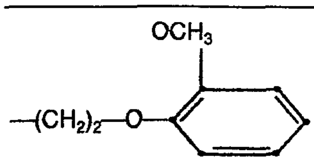 | H |

Sous forme de dichlorhydrate

1) Chez la souris

— Par voie intragastrique : $DL_{50} > 5\,000$ mg/kg

2) Chez le rat

— Par voie intraveineuse : $DL_{50} =$ 245 mg/kg.

Les compositions thérapeutiques selon l'invention peuvent être présentées sous toute forme convenant à l'administration en thérapie humaine ou vétérinaire. Pour ce qui concerne l'unité d'administration, celle-ci peut prendre la forme, par exemple, d'un comprimé, d'une dragée, d'une poudre, d'une capsule, d'une gélule, d'un sirop pour l'administration orale, d'un suppositoire pour l'administration rectale ou d'une solution ou suspension pour l'administration parentérale.

Suivant la voie d'administration choisie, les compositions thérapeutiques de l'invention seront préparées en associant au moins un dérivé de pyridoxine de formule I ou un sel d'addition non toxique de ce dérivé avec un excipient approprié, ce dernier pouvant être constitué par exemple d'au moins un ingrédient sélectionné parmi les substances suivantes : eau distillée, alcool benzylique, lactose, amidons, talc, stéarate de magnésium, polyvinylpyrrolidone, acide alginique, silice colloïdale ou agents édulcorants.

Les exemples non limitatifs suivants illustrent la préparation des composés de l'invention ainsi qu'une composition thérapeutique.

## Exemple 1

Dihydroxyméthyl-4,5[hydroxy-2(méthyl-2 phénoxyéthylamino)-3 propoxy]-3 méthyl-2 pyridine et ses sels

### a) O-Allyl-3 α,α-isopropylidènepyridoxol

Dans un ballon de 1 l muni d'un réfrigérant et d'un agitateur, on place 400 ml d'éthanol absolu auquel on ajoute, par fractions, 9,2 g (0,4 at. g) de sodium.

Dès la réaction terminée et après refroidissement, on ajoute 49,15 g (0,2 mole) d'α,α-isopropylidènepyridoxol puis 15,31 g (0,2 mole) de chlorure d'allyle.

Sous agitation, on porte la solution obtenue pendant 4 jours au reflux. On évapore sous vide la plus grande partie de l'éthanol et on reprend le résidu dans l'eau. On extrait le milieu avec de l'éther et on lave l'extrait plusieurs fois avec une solution aqueuse d'hydroxyde de sodium (environ 2,5 N) puis avec de l'eau.

Après séchage, on évapore l'éther et on utilise le résidu tel quel. De cette manière, on obtient 30 g de O-allyl-3 α,α-isopropylidènepyridoxol. Rendement : 60 %.

P.F. après recristallisation de l'oxalate dans l'acétate d'éthyle : 97-98 °C.

### b) O-(Epoxy-2,3 propyl)-3 α,α-isopropylidènepyridoxol

Dans un ballon de 1 l muni d'un agitateur, on introduit 200 ml d'éther éthylique, 49,8 g (0,2 mole) de O-allyl-3 α,α-isopropylidènepyridoxol, 36,6 g (0,2 mole) de N-bromosuccinimide et 200 ml d'eau. On agite le mélange, ainsi obtenu, pendant cinq jours à la température de 20 °C. Après neutralisation au bicarbonate de sodium, on filtre l'insoluble que l'on lave avec de l'eau et de l'éther éthylique. On obtient ainsi une première fraction de bromhydrine. On en obtient une seconde au départ de la solution éthérée qui est lavée, séchée et évaporée à sec sous vide. On reprend le résidu dans l'éther diisopropylique ou dans l'éther diéthylique, on le travaille et on le filtre. Après réunion des deux fractions, on obtient un mélange de 52 à 55 g lequel fond aux environs de 130 °C et présente deux taches en chromatographie sur couche mince. On met ce mélange en suspension dans 160 ml d'éther éthylique et l'on agite pendant 4 heures à 20 °C en présence de 160 ml d'une solution aqueuse normale d'hydroxyde de sodium. Après décantation, lavage et séchage, on évapore la phase éthérée à sec et sous vide (rendement : de 90 à 100 %). Le résidu est éventuellement cristallisé dans l'éther diisopropylique. De cette manière, on obtient le O-(époxy-2,3 propyl)-3 α,α-isopropylidènepyridoxol avec un rendement de cristallisation de 90 % environ.

P.F. : 76 °C.

### c) Dihydroxyméthyl-4,5[hydroxy-2(méthyl-2 phénoxyéthylamino)-3 propoxy]-3 méthyl-2 pyridine

Pendant 4 heures, on porte au reflux une solution de 795 g (3 moles) de O-(époxy-2,3 propyl)-3 α,α-isopropylidènepyridoxol et 453 g (3 moles) de O-méthylphénoxyéthylamine dans 250 ml de méthanol. Après cette opération, on évapore le milieu réactionnel sous vide.

On hydrolyse le résidu sous agitation d'abord à 20 °C pendant 12 heures en présence d'une solution de 700 ml d'acide chlorhydrique dans 2 500 ml d'eau puis pendant 30 minutes à 80 °C. Après refroidissement, on alcalinise le milieu par ajout de 700 g de carbonate de potassium puis on extrait d'abord avec un mélange de 2 000 ml/400 ml de chloroforme/n-butanol puis avec 500 ml de chloroforme. On réunit les

phases organiques, on les lave 3 fois avec de l'eau et on les sèche sur sulfate de sodium. Après filtration, on ajoute 2 000 ml de méthanol.

De cette manière, on obtient la dihydroxyméthyl-4,5[hydroxy-2(méthyl-2 phénoxyéthylamino)-3 propoxy]-3 méthyl-2 pyridine sous forme de base libre.

P.F. : environ 100 °C.

d) Sesquioxalate de dihydroxyméthyl-4,5[hydroxy-2(méthyl-2 phénoxyéthylamino)-3 propoxy]-3 méthyl-2 pyridine

A la base libre obtenue précédemment on ajoute 540 g d'acide oxalique anhydre dans 1 000 ml de méthanol. On filtre l'oxalate qui précipite et on le lave avec du méthanol.

Après trois cristallisations dans le méthanol, on obtient 380 g de sesquioxalate de dihydroxyméthyl-4,5[hydroxy-2(méthyl-2 phénoxyéthylamino)-3 propoxy]-3 méthyl-2 pyridine.

P.F. : 150-151 °C.

e) Monofumarate de dihydroxyméthyl-4,5[hydroxy-2(méthyl-2 phénoxyéthylamino)-3 propoxy]-3 méthyl-2 pyridine

Dans un ballon, on introduit 37,6 g (0,1 mole) de la base libre, obtenue au paragraphe c précédent, dissoute dans l'isopropanol. Après cette opération, on ajoute 23,2 g (0,2 mole) d'acide fumarique dans l'isopropanol. On porte le milieu au reflux et on le concentre. Après cette opération, on ajoute de l'acétate d'éthyle et on concentre à nouveau le milieu. Par refroidissement, une masse se forme que l'on reprend dans l'acétate d'éthyle. Après filtration, on cristallise le milieu dans un mélange acétate d'éthyle/méthanol.

De cette manière, on obtient le monofumarate de dihydroxyméthyl-4,5[hydroxy-2(méthyl-2 phénoxyéthylamino)-3 propoxy]-3 méthyl-2 pyridine.

P.F. : 119-120 °C (décomposition).

f) Dichlorhydrate de dihydroxyméthyl-4,5[hydroxy-2(méthyl-2 phénoxyéthylamino)-3 propoxy]-3 méthyl-2 pyridine

Dans un ballon, on introduit 37,6 g (0,1 mole) de base libre, obtenue au paragraphe c précédent, dissoute dans un mélange méthanol/éther éthylique. Après cette opération, on fait barboter de l'acide chlorhydrique sec et on ajoute de l'éther éthylique. On recristallise deux fois, dans un mélange acétate d'éthyle/méthanol, le précipité ainsi formé.

De cette manière, on obtient le dichlorhydrate de dihydroxyméthyl-4,5[hydroxy-2(méthyl-2 phénoxyéthylamino)-3 propoxy]-3 méthyl-2 pyridine.

P.F. : 151-152 °C (décomposition).

g) Dimaléate de dihydroxyméthyl-4,5[hydroxy-2(méthyl-2 phénoxyéthylamino)-3 propoxy]-3 méthyl-2 pyridine

Dans un ballon, on introduit 37,6 g de base libre, obtenue au paragraphe c précédent, dissoute dans du méthanol et on porte au reflux en présence de 23,2 g (0,2 mole) d'acide maléique. On porte le mélange à sec sous vide et on lave deux fois avec de l'éther éthylique sec. Après cette opération on cristallise le milieu dans un mélange acétone/acétate d'éthyle et on recristallise dans un mélange isopropanol/méthanol. De cette manière, on obtient le dimaléate de dihydroxyméthyl-4,5[hydroxy-2(méthyl-2 phénoxyéthylamino)-3 propoxy]-3 méthyl-2 pyridine.

P.F. : 113-116 °C.

h) Pamoate de dihydroxyméthyl-4,5[hydroxy-2(méthyl-2 phénoxyéthylamino)-3 propoxy]-3 méthyl-2 pyridine

Dans un ballon, on introduit 37,6 g (0,1 mole) de base libre, obtenue au paragraphe c précédent, dissoute dans du méthanol et on porte au reflux en présence de 38,8 g (0,1 mole) d'acide pamoïque. On évapore le mélange à sec et sous vide et on lave le résidu avec de l'éther éthylique sec. Après cette opération, on cristallise le milieu dans l'isopropanol et on recristallise dans un mélange méthanol/isopropanol.

De cette manière, on obtient le pamoate de dihydroxyméthyl-4,5[hydroxy-2(méthyl-2 phénoxyéthylamino)-3 propoxy]-3 méthyl-2 pyridine.

P.F. : 177-179 °C.

De la même manière que celle décrite ci-dessus, on a préparé les composés suivants :

(Voir tableau page suivante)

| Composé | P.F. °C |
|---|---|
| Dioxalate de dihydroxyméthyl-4,5(hydroxy-2 phénoxyéthylamino-3 propoxy)-3 méthyl-2 pyridine | 110-115 (méthanol) |
| Sesquioxalate de dihydroxyméthyl-4,5 [hydroxy-2(méthyl-3 phénoxyéthylamino)-3 propoxyl]-3 méthyl-2 pyridine | 168-169 (méthanol) |
| Sesquioxalate de dihydroxyméthyl-4,5 [(éthyl-2 phénoxyéthylamino)-3 hydroxy-2 propoxyl]-3 méthyl-2 pyridine | 149-151 (méthanol) |
| Sesquioxalate de dihydroxyméthyl-4,5 [(diméthyl-2,6 phénoxyéthylamino)-3 hydroxy-2 propoxyl]-3 méthyl-2 pyridine | 133-135 (méthanol) |
| Sesquioxalate de dihydroxyméthyl-4,5 [hydroxy-2(diméthyl-3,5 phénoxyéthylamino)-3 propoxy]-3 méthyl-2 pyridine | 178-179 (méthanol) |
| Sesquioxalate de dihydroxyméthyl-4,5 [hydroxy-2(méthoxy-2 phénoxyéthylamino)-3 propoxy]-3 méthyl-2 pyridine | 132-133 (méthanol) |
| Sesquioxalate de dihydroxyméthyl-4,5 [hydroxy-2(phénoxy-2 méthyl-2 éthylamino)-3 propoxyl]-3 méthyl-2 pyridine | 111-115 (méthanol) |
| Oxalate de dihydroxyméthyl-4,5 [hydroxy-2(méthyl-1 phénoxy-2 éthylamino)-3 propoxy]-3 méthyl-2 pyridine | 180-181 (isopropanol/méthanol) |
| Dioxalate de dihydroxyméthyl-4,5 [hydroxy-2(isopropyl-2 méthyl-5 phénoxyéthylamino)-3 propoxy]-3 méthyl-2 pyridine | 164-165 (méthanol) |
| Sesquioxalate de dihydroxyméthyl-4,5 [hydroxy-2(méthyl-4 phénoxyéthylamino)-3 propoxy]-3 méthyl-2 pyridine | 133-135 (méthanol) |
| Dioxalate de dihydroxyméthyl-4,5 [hydroxy-2(n-propyl-2 phénoxyéthylamino)-3 propoxyl]-3 méthyl-2 pyridine | 143-145 (méthanol) |
| Dioxalate de dihydroxyméthyl-4,5 [hydroxy-2(isopropyl-2 phénoxyéthylamino)-3 propoxy]-3 méthyl-2 pyridine | 165-166 (méthanol) |
| Sesquioxalate de dihydroxyméthyl-4,5 [hydroxy-2(diméthyl-3,4 phénoxyéthylamino)-3 propoxyl]-3 méthyl-2 pyridine | 184-185 (méthanol) |
| Dihydroxyméthyl-4,5 [hydroxy-2(isopropyl-2 phénoxyéthylamino)-3 propoxyl]-3 méthyl-2 pyridine | 102-103 (éther diisopropylique/méthanol) |
| Sesquioxalate de dihydroxyméthyl-4,5 [hydroxy-2(diméthyl-3,4 phénoxypropylamino)-3 propoxy]-3 méthyl-2 pyridine | 147-150 (éther diisopropylique/méthanol) |
| Dichlorhydrate de dihydroxyméthyl-4,5 [hydroxy-2(méthoxy-2 phénoxyéthylamino)-3 propoxy]-3 méthyl-2 pyridine | 107-110 (acétate d'éthyle/méthanol) |
| Sesquioxalate de dihydroxyméthyl-4,5 [hydroxy-2(chloro-4 phénoxyéthylamino)-3 propoxy]-3 méthyl-2 pyridine | 112-115 (méthanol) |

Exemple 2

On a préparé une unité d'administration orale en introduisant 300 mg de sesquioxalate de dihydroxyméthyl-4,5[hydroxy-2(méthyl-2 phénoxyéthylamino)-3 propoxy]-3 méthyl-2 pyridine dans une gélule.

**Revendications**

1. Dérivés de pyridoxine correspondant à la formule générale :

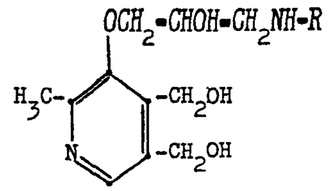

dans laquelle R représente un radical

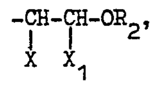, $-CH_2-(CH_2)_n-CH_2-OR_2$

dans lequel n représente 0 ou 1, X et $X_1$ qui sont différents, représentent hydrogène ou méthyle, et $R_2$ représente un groupement phényle non substitué ou substitué par un ou deux substituants sélectionnés du groupe formé par le fluor, le chlore et le brome et par les radicaux méthyle, éthyle, n-propyle, isopropyle ou méthoxy, ainsi que les sels d'addition non toxiques de ces dérivés.

2. Dérivés de pyridoxine selon la Revendication 1 dans laquelle X, $X_1$ et n ont la signification y citée et $R_2$ représente un groupement phényle non substitué ou substitué par un ou deux radicaux méthyle ou encore par un radical éthyl-2, n-propyl-2, isopropyl-2 ou méthoxy-2, diméthoxy-3,4, isopropyl-2 méthyl-5 ou chloro-4 ainsi que les sels d'addition non toxiques de ces composés.

3. Dihydroxyméthyl-4,5[hydroxy-2(méthyl-2 phénoxyéthylamino)-3 propoxy]-3 méthyl-2 pyridine et ses sels d'addition non toxiques.

4. Dihydroxyméthyl-4,5[hydroxy-2(méthoxy-2 phénoxyéthylamino)-3 propoxy]-3 méthyl-2 pyridine et ses sels d'addition non toxiques.

5. Dérivés de pyridoxine selon l'une quelconque des Revendications 1 à 4 sous la forme d'un sel d'addition non toxique obtenu à partir de l'acide chlorhydrique, l'acide oxalique, l'acide fumarique, l'acide maléique ou l'acide pamoïque.

6. Procédé de préparation de dérivés de pyridoxine selon la Revendication 1, caractérisé en ce que l'on condense, au reflux et dans un solvant approprié, une amine primaire de formule générale :

$$H_2N—R$$

dans laquelle R a la même signification que dans la Revendication 1 avec le O-(époxy-2,3 propyl)-3 α,α-isopropylidènepyridoxol de formule :

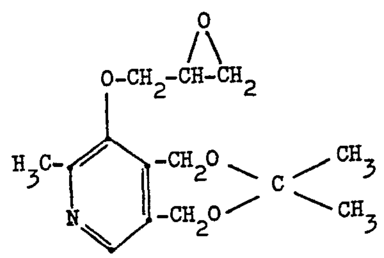

pour obtenir un cétal que l'on hydrolyse par la suite en milieu acide fort et à une température comprise entre 25 et 80 °C, ce qui fournit le dérivé désiré de pyridoxine, dans lequel $R_1$ représente hydrogène, sous la forme de base libre, la base libre obtenue étant par la suite mise en réaction, si on le désire, avec un acide organique ou inorganique approprié pour former un sel d'addition non toxique.

7. Procédé selon la Revendication 6, caractérisé en ce que le solvant est le méthanol et le milieu acide fort est l'acide chlorhydrique.

8. Procédé selon la Revendication 6, caractérisé en ce que l'on fait réagir la base libre avec l'acide chlorhydrique, l'acide oxalique, l'acide fumarique, l'acide maléique ou l'acide pamoïque pour obtenir un sel d'addition non toxique.

9. Composition pharmaceutique ou vétérinaire caractérisée en ce qu'elle comprend, comme principe actif, au moins un dérivé de pyridoxine selon l'une quelconque des Revendications 1, 2 ou 5, en association avec un véhicule pharmaceutique ou un excipient approprié.

10. Composition pharmaceutique ou vétérinaire caractérisée en ce qu'elle comprend, comme principe actif, un dérivé pyridoxine selon la Revendication 3 ou 4, en association avec un véhicule pharmaceutique ou un excipient approprié.

**Claims**

1. Pyridoxine derivatives represented by the general formula :

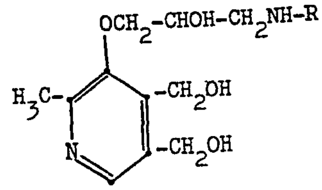

in which R represents a radical

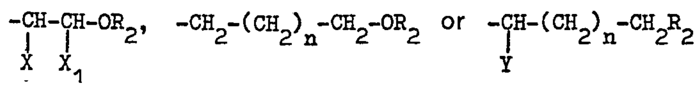

in which n represents 0 or 1, X and $X_1$, which are different, represent hydrogen or methyl, Y represents hydrogen or methyl and $R_2$ represents a phenyl group non-substituted or bearing one or two substituents selected from the group consisting of fluorine, chlorine and bromine and of the radicals methyl, ethyl, n-propyl, isopropyl and methoxy, as well as the pharmaceutically acceptable acid addition salts thereof.

2. Pyridoxine derivatives according to Claim 1, in which X, $X_1$, Y and n have the same meaning as given therein, $R_2$ represents a phenyl group non-substitued or bearing one or two substituents selected from the group consisting of methyl, 2-ethyl, 2-n-propyl, 2-isopropyl, 2-methoxy, 3,4-dimethyl, 3,4-dimethoxy, 2-isopropyl-5-methyl and 4-chloro as well as the pharmaceutically acceptable acid addition salts thereof.

3. 4,5-Dihydroxymethyl-3-[2-hydroxy-3-(2-methyl-phenoxyethylamino)-propoxy]-2-methyl-pyridine and pharmaceutically acceptable acid addition salts thereof.

4. 4,5-Dihydroxymethyl-3-[2-hydroxy-3-(2-methoxy-phenoxyethylamino)-propoxy]-2-methyl-pyridine and pharmaceutically acceptable acid addition salts thereof.

5. Pyridoxine derivatives according to any of Claims 1 to 4, in the form of a pharmaceutically acceptable acid addition salt obtained from hydrochloric acid, oxalic acid, fumaric acid, maleic acid or pamoic acid.

6. Process for preparing pyridoxine derivatives according to Claim 1, whereby a primary amine of general formula :

$$H_2N—R$$

in which R has the same meaning as in Claim 1, is condensed under reflux and in an appropriate solvent with 3-O-(2,3-epoxy-propyl)-α,α-isopropylidenepyridoxol of formula :

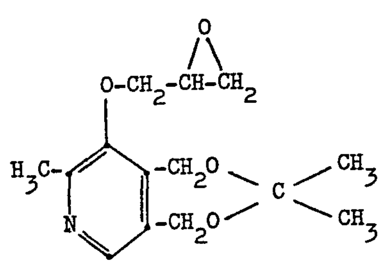

to obtain a ketal which is subsequently hydrolysed in a strong acid medium and at a temperature between 25 and 80 °C to provide the required pyridoxine derivative in the form of a free base, the free base obtained being further reacted, if desired, with an appropriate organic or inorganic acid to form a pharmaceutically acceptable acid addition salt.

7. Process according to Claim 6 wherein the solvent is methanol and the strong medium is hydrochloric acid.

8. Process according to Claim 6, wherein the free base is reacted with hydrochloric acid, oxalic acid, fumaric acid, maleic acid or pamoic acid to form a pharmaceutically acceptable acid addition salt.

9. A pharmaceutical or veterinary composition comprising as active ingredient at least one pyridoxine derivative according to any of Claim 1 to 5, in association with a pharmaceutical carrier or excipient therefor.

10. A pharmaceutical or veterinary composition comprising as active ingredient a pyridoxine derivative according to Claim 3 or 4, in association with a pharmaceutical carrier or excipient therefor.

**Ansprüche**

1. Pyridoxinderivate der allgemeinen Formel :

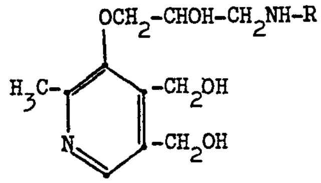

in der R einen Rest

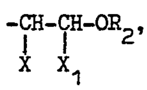, $-CH_2-(CH_2)_n-CH_2-OR_2$ oder 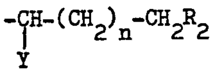

bedeutet, wobei n 0 oder 1 ist, X und $X_1$, die unterschiedlich sind, Wasserstoff oder Methyl darstellen, Y Wasserstoff oder Methyl bedeutet und $R_2$ eine Phenylgruppe darstellt, die unsubstituiert ist oder einen oder zwei Substituenten aus der Gruppe, bestehend aus Fluor, Chlor und Brom und den Resten Methyl, Ethyl, n-Propyl, Isopropyl und Methoxy, trägt sowie pharmazeutisch verträgliche Säureadditionssalze davon.

2. Pyridoxinderivate nach Anspruch 1, bei denen X, $X_1$, Y und n die dort angegebene Bedeutung haben, $R_2$ eine Phenylgruppe darstellt, die unsubstituiert ist oder einen oder zwei Substituenten aus der Gruppe, bestehend aus Methyl, 2-Ethyl, 2-n-Propyl, 2-Isopropyl, 2-Methoxy, 3-4-Dimethyl, 3,4-Dimethoxy, 2-Isopropyl-5-methyl und 4-Chlor, trägt sowie die pharmazeutisch verträglichen Säureadditionssalze davon.

3. 4,5-Dihydroxymethyl-3-[2-hydroxy-3-(2-methyl-phenoxyethylamino)-propoxy]-2-methyl-pyridin und die pharmazeutisch verträglichen Säureadditionssalze davon.

4. 4,5-Dihydroxymethyl-3-[2-hydroxy-3-(2-methoxy-phenoxyethylamino)-propoxy]-2-methyl-pyridin und die pharmazeutisch verträglichen Säureadditionssalze davon.

5. Pyridoxinderivate nach irgendeinem der Ansprüche 1 bis 4, in Form eines pharmazeutisch verträglichen Säureadditionssalzes, erhalten aus Chlorwasserstoffsäure, Oxalsäure, Fumarsäure, Maleinsäure oder Pamoasäure.

6. Verfahren zur herstellung von Pyridoxinderivaten nach Anspruch 1, wobei ein primäres Amin der allgemeinen Formel :

$$H_2N—R$$

in der R dieselbe Bedeutung wie in Anspruch 1 hat, unter Rückfluß in einem geeigneten Lösungsmittel mit 3-O-(2,3-Epoxy-propyl)-α,α-isopropylidenpyridoxol der Formel :

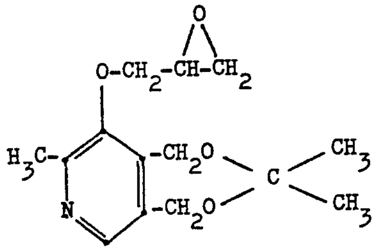

zu einem Ketal kondensiert wird, das anschließend in einem stark sauren Medium bei einer Temperatur zwischen 25 und 80 °C hydrolysiert wird, um das gewünschte Pyridoxinderivat in Form einer freien Base zu ergeben, wobei die erhaltene freie Base gegebenenfalls mit einer geeigneten organischen oder anorganischen Säure zu einem pharmazeutisch verträglichen Säureadditionssalz weiter umgesetzt wird.

7. Verfahren nach Anspruch 6, worin das Lösungsmittel Methanol und das stark saure Medium Salzsäure ist.

8. Verfahren nach Anspruch 6, worin die freie Base mit Chlorwasserstoffsäure, Oxalsäure, Fumarsäure, Maleinsäure oder Pamoasäure zu einem pharmazeutisch verträglichen Säureadditionssalz umgesetzt wird.

9. Pharmazeutische oder tiermedizinische Zusammensetzung, umfassend als Wirkstoff mindestens ein Pyridoxinderivat nach irgendeinem der Ansprüche 1 bis 5, zusammen mit einem pharmazeutischen Träger oder Excipienten dafür.

10. Pharmazeutische oder tiermedizinische Zusammensetzung, umfassend als Wirkstoff ein Pyridoxinderivat nach Anspruch 3 oder 4, zusammen mit einem pharmazeutischen Träger oder Excipienten dafür.